# EUROPEAN PATENT SPECIFICATION

(11) **EP 3 908 118 B1**
(45) Date of publication and mention of the grant of the patent: **14.05.2025**
(21) Application number: 20700118.1
(22) Date of filing: 09.01.2020
(51) Int. Cl.: A23B 7/148, A23B 7/152, A23B 7/144, F25D 13/00

(54) **APPARATUS AND METHOD FOR CONTROLLING THE STATE OF PRESERVATION OF FOODSTUFFS**
VORRICHTUNG UND VERFAHREN ZUR KONTROLLE DES KONSERVIERUNGSZUSTANDES VON LEBENSMITTELN
APPAREIL ET METHODE DE CONTROLE DE L'ETAT DE CONSERVATION DE PRODUITS ALIMENTAIRES

(30) Priority: 11.01.2019 IT 201900000448
(43) Date of publication of application: 17.11.2021
(73) Proprietor: Aria S.r.l., 39100 Bolzano (IT)
(72) Inventor: PRUNERI, Marco, 39044 EGNA (BOLZANO) (IT)
(74) Representative: Forattini, Amelia
(86) International application number: PCT/EP2020/050385
(87) International publication number: WO 2020/144256

(56) References cited:
- WO-A1-2014/066952
- WO-A1-2016/015167
- WO-A1-2017/035554
- US-A- 5 822 068
- US-A1- 2018 310 579

## Description

The present invention relates to an apparatus and a method for controlling the state of preservation of foodstuffs.

The long-term preservation of most varieties of fresh fruit and of some varieties of vegetables is performed in cold storage units which operate above the freezing temperature of the varieties to be preserved and are known in the technical jargon as "0°C units", an expression that intends to indicate that these are not low-temperature (< 0°C) cold storage units for preservation of frozen foodstuffs.

Typically, these cold storage units, in addition to being equipped with an appropriately sized refrigeration system, are also equipped with an apparatus for controling the internal atmosphere, which in the technical jargon is referenced as "CA system".

The CA system is meant to extend the preservation of the fresh fruit and vegetables for periods even three times longer than traditional preservation in an ordinary cold storage unit.

This result is achieved by controlling and maintaining the optimum specific values, for each variety to be preserved, of oxygen, carbon dioxide, relative humidity, and in some specific cases also of ethylene, throughout the period of preservation in the cold storage unit.

The most recent and sophisticated method for the control and adjustment of these atmospheric preservation values, which has been developed and has become established in recent years, is known as "dynamic atmosphere", or to use the English acronym, "DCA", which stands for "Dynamic Controlled Atmosphere".

The use of DCA has allowed a further significant improvement in the quality of the preservation of fresh fruit and vegetables, increasing the time limits of preservability of the varieties preserved with this method and improving the results of long-term preservation.

Currently there are three methods of application of DCA, all three of which are characterized by an initial step in which the level of oxygen is lowered in the preservation cell below the level that is physiologically tolerable by the horticultural variety being preserved, known in the technical jargon as "stress level", by maintaining this stress level for a very specific period of time, and by restoring the level of oxygen to a value that is tolerable and is compatible with the life of the variety.

Essentially, all three methods require establishing in the CA cell asphyxial conditions, which are not tolerable or lethal for the horticultural variety being preserved, measuring the progressive increase in stress induced by these conditions, and interrupting at an appropriate time this stress condition.

The differences between the three systems consist of the methods for monitoring and evaluating the stress level reached by the variety being preserved and the stress period to be maintained.

The first, oldest and least refined of the three methods is known as ILOS (acronym of Initial Low Oxygen Stress).

ILOS measures the stress level by measuring the quantity of ethanol, a particular alcohol that is created inside the pulp, during this step. This ethanol measurement is performed by means of destructive chemical measurements.

In practice, this measurement is performed by taking samples of the fruit and vegetables to be analyzed from the cell, which must then be taken to a laboratory site, equipped to measure the quantities of ethanol/alcohol that are present in the variety, where this type of analysis will indeed be performed.

Of the three methods, this is certainly the least refined and most approximate, since in addition to being the only one to use destructive measurement methods it is also the one with the lowest analysis resolution and which, by requiring the manual sampling and processing of most of the analysis procedure, is also the one with the lowest sampling rate between one measurement and the next.

It is clearly evident that in this method the intervals between the various analyses on the samples are necessarily rather large, since the method, which is off-line, indeed entails that every time one wishes to perform ethanol analysis in the variety under stress it is necessary to take samples from the cold storage unit, take them to the site equipped for analysis and then perform the analysis.

The second and third methods instead provide for online, real-time and completely automated analyses, which are performed directly in the preservation cell.

The second method is termed "respiratory quotient analysis" it is based on the premise or fact that the variety being stressed changes its "way of breathing".

In practice, during their life and during a normal preservation in the cold storage unit or in a controlled atmosphere, vegetables consume oxygen and produce a substantially proportional and equivalent quantity of carbon dioxide.

When they are subjected to stress, instead, they decrease drastically their oxygen consumption and greatly increase the quantity of emitted carbon dioxide.

This method is based on the measurement and verification of the trend of the oxygen and carbon dioxide values that are present in the cell. It is performed continuously and is therefore capable of detecting more finely the variation of the induced stress.

The main problem of this method resides in the requirement of a perfect hermetic seal required of the preservation cell. Any slightest entry of air from the outside is in fact potentially capable of diluting the concentrations of the gas mixture in the cell and accordingly the validity of the analysis of the respiratory quotient.

Unfortunately, the requirement of a perfect hermetic seal is very difficult to provide, due to the large dimensions of the systems and of the cold storage units in which fresh fruit is preserved. This is the reason why this method currently is used only in the laboratory and is not applied in refrigerated storage units, which are practice large industrial facilities.

The third analysis is based on the fact that the stress induced on the vegetables also induces a change in their photosynthesis activity.

Photosynthesis activity is measured currently by means of optical sensors, which measure the luminescence response induced in fruit by radiating light thereon.

This method is currently the most advanced of the three previously described here and is also the only one that is currently automated and used at a worldwide level also outside laboratories, in large CA storage units.

Despite the undeniable advantages that the DCA method has provided in the field of fresh fruit and vegetables preservation, currently there is no system that is completely automated and operates continuously in the field and is capable of monitoring in real time the state of preservation of the stored fruit and vegetables and of providing information that allows to manage and optimize the process for preserving and marketing the stored product.

US2018/310579A1 discloses a system for controlling low temperature injury of cold-sensitive fruit vegetables by combining intelligent pre-cooling and segmented controlled atmosphere storage. The system includes a sensor detection module, a data collection module, a wireless communications module, a terminal, and a control system, where the sensor detection module detects carbon dioxide, ethylene, and the temperature and the humidity within a fresh keeping chamber, then data is collected by using the data collection module connected to the sensor detection module, and then varying data is transmitted to the terminal by using the wireless communications module connected to the data collection module. The sensor detection module is connected to the control system; after the terminal reads data detected by a sensor, an environment parameter is controlled by using the control system.

WO2017/035554A1 discloses a method for monitoring produce ripening. The method comprises receiving readings from a monitor module wherein the received readings are from a plurality of transmitters connected to the monitor module. The transmitters comprise an ethylene transmitter, a carbon dioxide transmitter, a humidity transmitter, an oxygen transmitter, and an ambient temperature transmitter. The transmitters provide the received readings to a controller. The method further comprises controlling produce ripening by receiving at the controller target readings and sending the target readings to the control module which controls a heater, a cooler and a gas delivery module. The gas delivery module has an ethylene source and the control module controls the output of the heater, the cooler and the gas delivery module to reach the target reading in the produce environment to thereby control the produce ripening.

WO2014/066952A1 discloses a method of controlling gas composition within a container containing respiring produce. The container includes a gas outlet and a gas inlet and the method includes the steps of; drawing a selected gas from within the container through a selective membrane element and through the gas outlet; detecting ambient pressure inside the container; and introducing ambient air under selected control conditions from outside the container into the container through the gas inlet, in response to the detected ambient pressure to control the relative composition of gases inside the container.

WO2016/015167A1 discloses a portable electronic device comprising sensors for measuring ambient and atmospheric parameters, a battery, and an information storage modal, inter alia, which allows the measurement, recording, transmission and/or storage of the measured ambient parameters.

US5822068A discloses a non-destructive method of testing fruits and vegetables for post-harvest quality, using fluorescence intensity of the skin or leaves. A low intensity red light source is used to irradiate the skin or leaves of fruits or vegetables to provide a first level Fo of fluorescence intensity above that of red light in the 710 to 740 nM range. A second high intensity red light source is used to produce a maximal second fluorescence intensity Fm in the skin or leaves in the 710 to 740 nM range. The ratio Fv/Fm=(Fm-Fo)/Fm is then determined to provide a measure of the quality of the fruit or vegetable.

The aim of the present invention is to provide an apparatus that overcomes the drawbacks of the cited background art.

Within the scope of this aim, a particular object of the invention is to provide an apparatus that allows to control the state of preservation of fruit and vegetables in an optimum manner and is suitable for industrial use.

Another important object of the invention is to provide an apparatus that allows to control the state of preservation of fruit and vegetables in a completely automated manner.

Another object of the present invention is to provide an apparatus which, by virtue of its particular constructive characteristics, is capable of giving the greatest assurances of reliability and safety in use.

This aim and these and other objects which will become better apparent hereinafter are achieved by an apparatus and a method for controlling the state of preservation of foodstuffs, as claimed in the appended claims.

Namely, the present invention relates to:
- An apparatus for controlling the state of preservation of foodstuffs, comprising a central data processing unit which collects, stores and displays information generated by a plurality of peripheral boards and transmitted to said central unit via field bus; said peripheral boards being arranged in a controlled-atmosphere environment which contains foodstuffs; each peripheral board comprising a sensor array and performing continuous measurements of various parameters that are characteristic of said foodstuffs; said apparatus being characterized in that each said peripheral board comprises a series of sensors comprising: a sensor adapted to measure the current value of the photosynthetic activity and capacity of a foodstuff; a sensor adapted to measure the current color of said foodstuff; a sensor adapted to measure the consistency and water quantity of said foodstuff; a sensor adapted to measure the temperature of said foodstuff and of said peripheral board; and a sensor adapted to measure the absolute and relative humidity of said peripheral board.
- A method for controlling the state of preservation of foodstuffs, characterized in that it comprises the collection, preservation and display of information transmitted by peripheral boards arranged in a controlled-atmosphere environment which contains a foodstuff; each one of said peripheral boards continuously monitoring various parameters of said foodstuff, at a suitable frequency and automatically; said parameters comprising: a current value of the photosynthesis activity and capacity of the foodstuff; a current color of the foodstuff; a measurement of the consistency and consequently of the water quantity of the foodstuff; and a measurement of the temperature of the body of the foodstuff and of the peripheral board; a measurement of the absolute and relative humidity of said board.

Further characteristics and advantages will become better apparent from the description of preferred but not exclusive embodiments of the invention, illustrated by way of nonlimiting example in the accompanying drawings, wherein:
Figure 1 is a block diagram of the apparatus according to the present invention;
Figure 2 is a plan view which shows schematically a practical embodiment of a peripheral board of the apparatus according to the invention;
Figure 3 is a schematic side view of the peripheral board of the preceding figure.

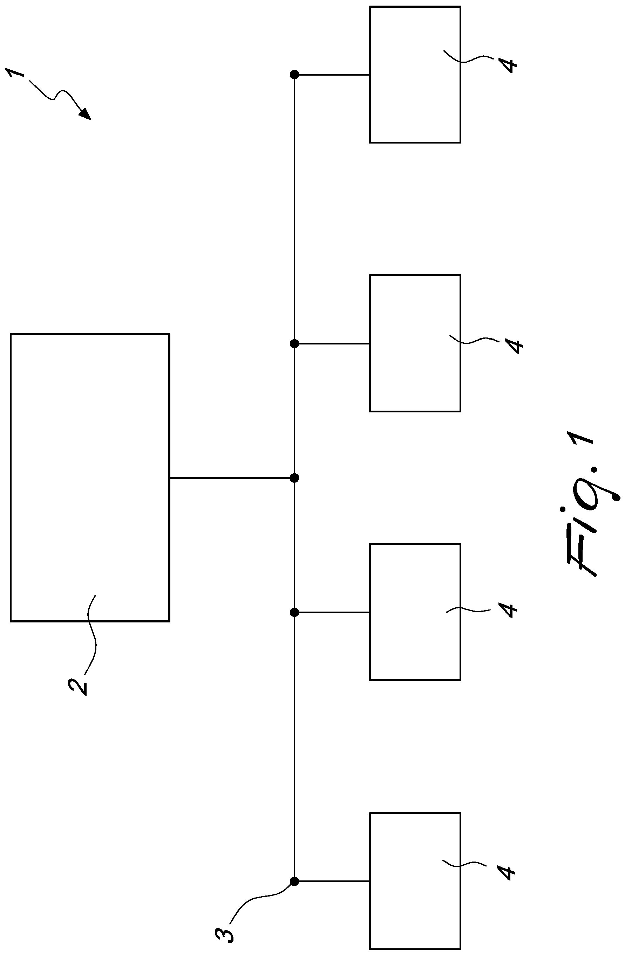

With particular reference to Figure 1, the apparatus according to the invention, designated generally by the reference numeral 1, includes a central data processing unit, designated by the reference numeral 2, which is designed to collect, preserve and display the information, transmitted via a field bus 3, by peripheral boards 4 which are arranged in the environment that contains foodstuffs, such as fruit or vegetables 5.

Each peripheral board 4, hereinafter designated by the term "DCA sensor" for the sake of simplicity, is constituted by electronic circuits which provide an array of sensors and transducers.

Every DCA sensor 4, which provides an array of sensors and transducers, is driven by a dedicated microcontroller and monitors in real time and continuously various characteristic parameters of the vegetable being preserved.

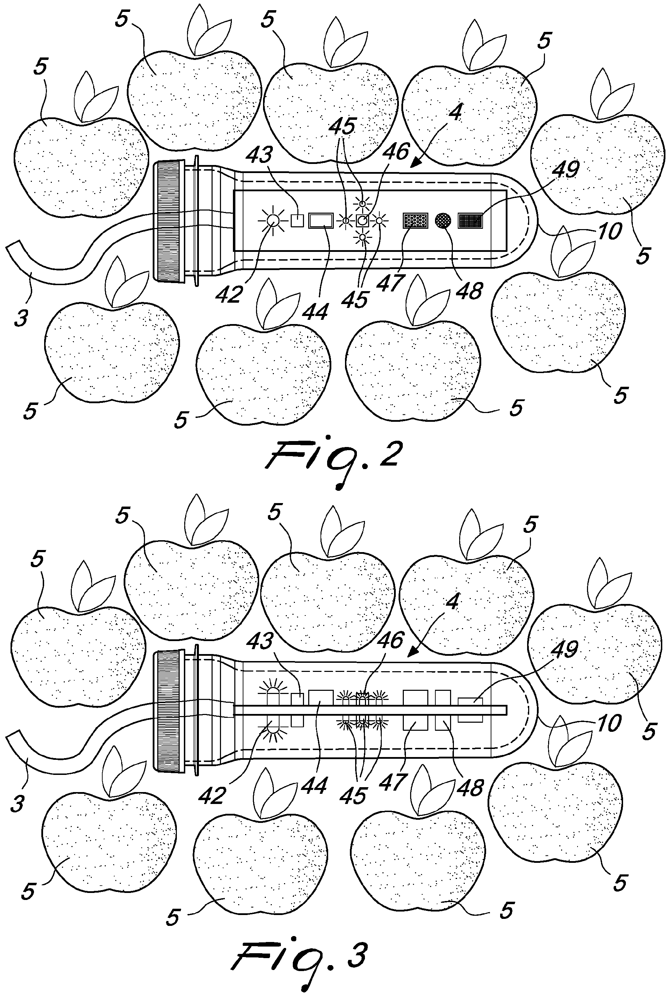

A constructive example of a peripheral board 4, DCA sensor, which provides the sensor array is shown in Figures 2 and 3.

The array of sensors that constitutes the core of each one of the peripheral boards 4 is capable of monitoring in real time the photosynthesis activity of the fruit and vegetables being preserved, the ripening process thereof, the quantity of water (juiciness) that is present and consequently also the consistency, in addition to the temperature of the body of the preserved variety.

By means of this information provided by the array of sensors, obtained continuously and automatically and in real time, the people assigned to the management of the preservation apparatus can both perform optimizations of the preservation parameters and make choices as to the best time to interrupt the long-term preservation process and to market the preserved products.

Likewise, by means of this information it is possible to highlight early the onset of problems in the preservation process and/or the possible pathologies that might occur in the varieties preserved for the most disparate reasons.

Deviation of the color, juiciness, consistency and photosynthesis activity of the variety being preserved with respect to what should be the standard ripening curves in the preservation period in fact allows to diagnose early even the slightest problem.

Advantageously, the central processing unit 2 of the apparatus according to the present invention communicates with the peripheral analysis boards 4, i.e., the DCA sensors, via field buses 3 constituted for example by RS-485, CAN buses, LIN buses.

The same cable formed by multiple wires that acts as a field and communication bus also provides the power supply to the various DCA sensors 4 included in the monitoring and control system/apparatus 1.

The central control system 2 collects the information from each DCA sensor 4, which in turn includes a sensor array.

The central control system 2 allows to display, both in a numeric table format and in graphical form, the current state and the evolution over time of each parameter measured by each DCA sensor 4.

The control system 2 can also be placed in communication with a web server which allows to verify, even via the Internet, the results and the state of the analyses of each DCA sensor 4.

The central control system 2 is provided by means of the combination of hardware dedicated to calculation and of a software program, which, by way of nonlimiting example, can be provided in the hardware part by means of a personal computer provided with a suitable operating system (for example Microsoft Windows, Apple MacOS, or Linux) or by means of specifically conceived and developed proprietary hardware.

The parameters measured by each DCA sensor 4, i.e., by each sensor array, are the following:
A) current value of the photosynthesis activity and capacity of the variety being preserved;
B) current color of the variety being preserved;
C) measurement of the consistency and accordingly of the quantity of water of the variety being preserved;
D) measurement of the temperature of the body of the variety being preserved and of the enclosure of the printed circuit of the sensor array;
E) measurement of the absolute and relative humidity inside the enclosure of the sensor array.

Each DCA sensor 4 contains a double, and therefore redundant, sensor array, each provided on each one of the two faces of a printed circuit board, as exemplified in Figures 2 and 3.

Each DCA sensor 4 is provided with a microcontroller 44 which manages the sensors and transducers, performs a first processing of the information obtained from the sensor array, and communicates the data to the central control unit.

Each peripheral board potentially exposed in the CA cells to temperature values that may vary between -5°C and +40°C, and to relative humidity values which are potentially close to 100%, is protected by a hermetic protective enclosure 10, preferably made of transparent plastic material.

The temperature of the environment and the relative and absolute humidity content are measured and monitored inside each hermetic protective enclosure 10 of each peripheral board 4 in order to alert the central system as to the potential imminence of condensation phenomena on the printed circuit, which potentially might compromise the operation of the sensors and of the electronic circuits.

By way of nonexhaustive example, the apparatus according to the invention performs the following measurements by means of each DCA sensor 4:
- a measurement of the photosynthetic activity, by using an appropriate lighting system, provided by means of LEDs or lamps 45 and a photodiode 46 equipped with an appropriate bandpass filter;
- a measurement of the quantity of water and of the consistency, by means of the emission of appropriate sounds of various tones/frequencies by means of a buzzer or, as an alternative, a micro-loudspeaker, designated by the reference numeral 48, and the subsequent measurement of the attenuation over time of the emitted sounds, performed by means of a microphone 49 of suitable sensitivity;
- a measurement of the color and of the color variations over time of the varieties being preserved by means of the lighting, performed with broad-spectrum lamps 42 or broad CRI diodes, and the measurement of the color by means of an appropriate color sensor 43;
- a temperature measurement by means of a temperature sensor 47;
- an absolute and relative humidity measurement, by means of a relative and absolute humidity sensor 47.

In practice it has been found that the invention achieves the intended aim and objects, providing an apparatus for controlling the state of preservation of foodstuffs, particularly of fresh fruit and vegetables, which provides a system that is completely automated and operates continuously in the field, allowing to monitor in real time the state of preservation of the stored fruit and vegetables and to provide information that allows to manage and optimize the process for preservation and marketing of the stored product.

## Claims

1. An apparatus for controlling the state of preservation of foodstuffs, comprising a central data processing unit (2) which collects, stores and displays information generated by a plurality of peripheral boards (4) and transmitted to said central unit (2) via field bus (3); said peripheral boards (4) being arranged in a controlled-atmosphere environment which contains foodstuffs; each peripheral board (4) comprising a sensor array and performing continuous measurements of various parameters that are characteristic of said foodstuffs; said apparatus being **characterized in that** each said peripheral board (4) comprises a series of sensors comprising: a sensor adapted to measure the current value of the photosynthetic activity and capacity of a foodstuff; a sensor adapted to measure the current color of said foodstuff; a sensor adapted to measure the consistency and water quantity of said foodstuff; a sensor adapted to measure the temperature of said foodstuff and of said peripheral board (4); and a sensor adapted to measure the absolute and relative humidity of said peripheral board (4).

2. The apparatus according to claim 1, **characterized in that** each said peripheral board (4) comprises a double, and therefore redundant, sensor array, each provided on each face of a printed circuit board.

3. The apparatus according to claim 1, **characterized in that** each said peripheral board (4) is contained in a hermetic protective enclosure (10).

4. The apparatus according to claim 1, **characterized in that** each said peripheral board (4) comprises at least one sensor for measuring photosynthetic activity which comprises a lighting system constituted by an LED or by lamps (45) and by a photodiode (46) provided with a bandpass filter.

5. The apparatus according to claim 1, **characterized in that** each said peripheral board (4) comprises at least one sensor for measuring the quantity of water and the consistency of said foodstuff, said sensor comprising a buzzer or loudspeaker (48) which emits sounds of various tones/frequencies and a microphone, said sensor measuring the attenuation over time of said emitted sounds.

6. The apparatus according to claim 1, **characterized in that** each said peripheral board (4) comprises at least one sensor for measuring the color and color variations over time of said foodstuff; said sensor comprising broad-spectrum lamps (42), or broad CRI diodes, which perform the lighting, and a color sensor which performs the color measurement.

7. The apparatus according to claim 1, **characterized in that** each said peripheral board (4) comprises at least one temperature sensor (47).

8. The apparatus according to claim 1, **characterized in that** each said peripheral board (4) comprises at least one absolute and relative humidity measurement sensor (47).

9. A method for controlling the state of preservation of foodstuffs, **characterized in that** it comprises the collection, preservation and display of information transmitted by peripheral boards arranged in a controlled-atmosphere environment which contains a foodstuff; each one of said peripheral boards continuously monitoring various parameters of said foodstuff, at a suitable frequency and automatically; said parameters comprising: a current value of the photosynthesis activity and capacity of the foodstuff; a current color of the foodstuff; a measurement of the consistency and consequently of the water quantity of the foodstuff; a measurement of the temperature of the body of the foodstuff and of the peripheral board; and a measurement of the absolute and relative humidity of said board.

## Patentansprüche

1. Eine Vorrichtung zur Kontrolle des Konservierungszustandes von Lebensmitteln, die eine zentrale Datenverarbeitungseinheit (2) umfasst, welche Informationen sammelt, speichert und anzeigt, die von einer Vielzahl peripherer Leiterplatten (4) erzeugt und über einen Feldbus (3) an die zentrale Einheit (2) gesendet wurden; wobei die peripheren Leiterplatten (4) in einer Umgebung mit kontrollierter Atmosphäre angeordnet sind, die Lebensmittel enthält; wobei jede periphere Leiterplatte (4) ein Sensorfeld umfasst und kontinuierliche Messungen verschiedener Parameter vornimmt, die charakteristisch für die Lebensmittel sind; wobei die Vorrichtung **dadurch gekennzeichnet ist, dass** jede periphere Leiterplatte (4) eine Reihe von Sensoren umfasst, die Folgendes umfassen: einen Sensor, ausgebildet, um den aktuellen Wert der Photosynthese-Aktivität und -Fähigkeit eines Lebensmittels zu messen; einen Sensor, ausgebildet, um die aktuelle Farbe des Lebensmittels zu messen; einen Sensor, ausgebildet, um die Konsistenz und den Wassergehalt des Lebensmittels zu messen; einen Sensor, ausgebildet, um die Temperatur des Lebensmittels und der peripheren Leiterplatte (4) zu messen; und einen Sensor, ausgebildet, um die absolute und relative Feuchte der peripheren Leiterplatte (4) zu messen.

2. Die Vorrichtung gemäß Anspruch 1, **dadurch gekennzeichnet, dass** jede periphere Leiterplatte (4) ein doppeltes, und somit redundantes, Sensorfeld umfasst, jeweils auf jeder Seite einer Leiterplatte angebracht.

3. Die Vorrichtung gemäß Anspruch 1, **dadurch gekennzeichnet, dass** jede periphere Leiterplatte (4) in einem hermetischen Schutzgehäuse (10) untergebracht ist.

4. Die Vorrichtung gemäß Anspruch 1, **dadurch gekennzeichnet, dass** jede periphere Leiterplatte (4) mindestens einen Sensor zum Messen der Photosynthese-Aktivität umfasst, der ein Beleuchtungssystem umfasst, bestehend aus einer LED oder aus Lampen (45), und aus einer Photodiode (46), die mit einem Bandpassfilter ausgestattet ist.

5. Die Vorrichtung gemäß Anspruch 1, **dadurch gekennzeichnet, dass** jede periphere Leiterplatte (4) mindestens einen Sensor zum Messen des Wassergehalts und der Konsistenz des Lebensmittels umfasst, wobei der Sensor einen Summer oder Lautsprecher (48) umfasst, welcher Geräusche mit verschiedenen Tonhöhen/Frequenzen emittiert, und ein Mikrofon, wobei der Sensor die Dämpfung der emittierten Geräusche über die Zeit misst.

6. Die Vorrichtung gemäß Anspruch 1, **dadurch gekennzeichnet, dass** jede periphere Leiterplatte (4) mindestens einen Sensor zum Messen der Farbe und der Farbveränderungen des Lebensmittels über die Zeit misst; wobei der Sensor Lampen (42) mit großem Spektralbereich oder Breit-CRI-Dioden umfasst, die die Beleuchtung vornehmen, und einen Farbsensor, der die Farbmessung durchführt.

7. Die Vorrichtung gemäß Anspruch 1, **dadurch gekennzeichnet, dass** jede periphere Leiterplatte (4) mindestens einen Temperatursensor (47) umfasst.

8. Die Vorrichtung gemäß Anspruch 1, **dadurch gekennzeichnet, dass** jede periphere Leiterplatte (4) mindestens einen Sensor (47) zur Messung der absoluten und relativen Feuchte umfasst.

9. Ein Verfahren zur Kontrolle des Konservierungszustands von Lebensmitteln, **dadurch gekennzeichnet, dass** es das Sammeln, Aufbewahren und Anzeigen von Informationen umfasst, die von peripheren Leiterplatten übertragen werden, welche in einer Umgebung mit kontrollierter Atmosphäre angeordnet sind, die ein Lebensmittel enthält; wobei jede der peripheren Leiterplatten kontinuierlich verschiedene Parameter des Lebensmittels mit einer geeigneten Frequenz automatisch überwacht; wobei die Parameter Folgendes umfassen: einen aktuellen Wert der Photosynthese-Aktivität und -Fähigkeit des Lebensmittels; eine aktuelle Farbe des Lebensmittels; eine Messung der Konsistenz und folglich des Wassergehalts des Lebensmittels; eine Messung der Temperatur des Körpers des Lebensmittels und der peripheren Leiterplatte und eine Messung der absoluten und relativen Feuchte der Leiterplatte.

## Revendications

1. Un appareil de contrôle de l'état de conservation de produits alimentaires, comprenant une unité centrale de traitement des données (2) qui collecte, stocke et affiche des informations générées par une pluralité de cartes périphériques (4) et transmises à ladite unité centrale (2) via un bus de terrain (3); lesdites cartes périphériques (4) étant disposées dans un environnement à atmosphère contrôlée qui contient des produits alimentaires; chaque carte périphérique (4) comprenant un réseau de capteurs et effectuant des mesures continues de divers paramètres qui sont caractéristiques desdites produits alimentaires; ledit appareil étant **caractérisé en ce que** chaque dite carte périphérique (4) comprend une série de capteurs comprenant: un capteur adapté à la mesure de la valeur courante de l'activité et de la capacité photosynthétique d'un produit alimentaire; un capteur adapté à la mesure de la couleur courante dudit produit alimentaire; un capteur adapté à la mesure de la consistance et de la quantité d'eau dudit produit alimentaire; un capteur adapté à la mesure de la température dudit produit alimentaire et de ladite carte périphérique (4); et un capteur adapté à la mesure de l'humidité absolue et relative de ladite carte périphérique (4).

2. L'appareil selon la revendication 1, **caractérisé par le fait que** chaque carte périphérique (4) comprend un double réseau de capteurs, donc redondant, chacun étant placé sur chaque face d'une carte de circuit imprimé.

3. L'appareil selon la revendication 1, **caractérisé par le fait que** chaque carte périphérique (4) est contenue dans un boîtier de protection hermétique (10).

4. L'appareil selon la revendication 1, **caractérisé en ce que** chaque dite carte périphérique (4) comporte au moins un capteur de mesure de l'activité photosynthétique qui comporte un système d'éclairage constitué par une LED ou par des lampes (45) et par une photodiode (46) munie d'un filtre passe-bande.

5. L'appareil selon la revendication 1, **caractérisé en ce que** chaque carte périphérique (4) comprend au moins un capteur pour mesurer la quantité d'eau et la consistance de ladite produit alimentaire, ledit capteur comprenant un buzzer ou haut-parleur (48) qui émet des sons de différentes tonalités/fréquences et un microphone, ledit capteur mesurant l'atténuation dans le temps des sons émis.

6. L'appareil selon la revendication 1, **caractérisé en ce que** chaque carte périphérique (4) comprend au moins un capteur pour mesurer la couleur et les variations de couleur dans le temps de ladite produit alimentaire; ledit capteur comprend des lampes à large spectre (42), ou des diodes à large IRC, qui effectuent l'éclairage, et un capteur de couleur qui effectue la mesure de la couleur.

7. L'appareil selon la revendication 1, **caractérisé en ce que** chaque carte périphérique (4) comprend au moins un capteur de température (47).

8. L'appareil selon la revendication 1, **caractérisé en ce que** chaque carte périphérique (4) comprend au moins un capteur de mesure d'humidité absolue et relative (47).

9. Procédé de contrôle de l'état de conservation de produits alimentaires, **caractérisé en ce qu'**il comprend la collecte, la conservation et l'affichage d'informations transmises par des cartes périphériques disposées dans un environnement à atmosphère contrôlée contenant une produit alimentaire; chacune de ces cartes périphériques surveillant en permanence divers paramètres de ladite produit alimentaire, à une fréquence appropriée et de manière automatique; ces paramètres comprenant: une valeur courante de l'activité et de la capacité de photosynthèse de produit alimentaire; une couleur courante de produit alimentaire; une mesure de la consistance et par conséquent de la quantité d'eau de produit alimentaire; une mesure de la température du corps de produit alimentaire et de la carte périphérique; et une mesure de l'humidité absolue et relative de ladite carte.
